(19) Europäisches Patentamt
European Patent Office
Office européen des brevets

(11) **EP 4 221 594 B1**

(12) **EUROPEAN PATENT SPECIFICATION**

(45) Date of publication and mention
of the grant of the patent:
**28.01.2026  Bulletin 2026/05**

(21) Application number: **21798221.4**

(22) Date of filing: **29.09.2021**

(51) International Patent Classification (IPC):
*A61B 5/367* (2021.01)     *A61B 5/287* (2021.01)
*A61B 5/349* (2021.01)     *A61B 5/00* (2006.01)

(52) Cooperative Patent Classification (CPC):
**A61B 5/367; A61B 5/287; A61B 5/349;
A61B 5/6852; A61B 5/6869**

(86) International application number:
**PCT/US2021/052586**

(87) International publication number:
**WO 2022/072453 (07.04.2022 Gazette 2022/14)**

(54) **ELECTROPHYSIOLOGY SYSTEM AND METHOD FOR IDENTIFYING SIGNIFICANT ELECTROGRAMS**

ELEKTROPHYSIOLOGIESYSTEM UND VERFAHREN ZUR IDENTIFIZIERUNG SIGNIFIKANTER ELEKTROGRAMME

SYSTÈME D'ÉLECTROPHYSIOLOGIE ET PROCÉDÉ POUR IDENTIFIER DES ÉLECTROCARDIOGRAMMES PERTINENTS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.09.2020  US 202063085671 P**

(43) Date of publication of application:
**09.08.2023  Bulletin 2023/32**

(73) Proprietor: **Boston Scientific Scimed Inc.
Maple Grove, Minnesota 55311 (US)**

(72) Inventors:
• **BENNETT, Nathan H.
Cambridge, Massachusetts 02138 (US)**
• **GELB-BICKNELL, Rudy
Somerville, Massachusetts 02144 (US)**

(74) Representative: **Pfenning, Meinig & Partner mbB
Patent- und Rechtsanwälte
Joachimsthaler Straße 10-12
10719 Berlin (DE)**

(56) References cited:
**US-A1- 2012 184 863     US-A1- 2018 296 108
US-A1- 2018 325 401**

## Description

TECHNICAL FIELD

The present disclosure relates to electrophysiology systems and methods for processing cardiac electrical signals.

BACKGROUND

Use of minimally invasive procedures, such as catheter ablation, to treat a variety of heart conditions, such as supraventricular and ventricular arrhythmias, is becoming increasingly more prevalent. Such procedures involve the mapping of electrical activity in the heart (e.g., based on cardiac signals), such as at various locations on the endocardium surface ("cardiac mapping"), to identify the site of origin of the arrhythmia followed by a targeted ablation of the site. To perform such cardiac mapping, a catheter with one or more electrodes can be inserted into the patient's heart chamber,

Conventional three-dimensional (3D) mapping techniques include contact mapping, non-contact mapping, and a combination of contact and non-contact mapping. In both contact and non-contact mapping, one or more catheters are advanced into the heart. With some catheters, once in the chamber, the catheter may be deployed to assume a 3D shape. In contact mapping, physiological signals resulting from the electrical activity of the heart are acquired with one or more electrodes located at the catheter distal tip after determining that the tip is in stable and steady contact with the endocardium surface of a particular heart chamber. In non-contact-based mapping systems, using the signals detected by the non-contact electrodes and information on chamber anatomy and relative electrode location, the system provides physiological information regarding the endocardium of the heart chamber. Location and electrical activity are usually measured sequentially on a point-by-point basis at about 50 to 200 points on the internal surface of the heart to construct an electro-anatomical depiction of the heart. The generated map may then serve as the basis for deciding on a therapeutic course of action, for example, tissue ablation, to alter the propagation of the heart's electrical activity and to restore normal heart rhythm.

In many conventional mapping systems, the clinician visually inspects or examines the captured electrograms (EGMs), which increases examination time and cost. During an automatic electro-anatomical mapping process, however, approximately 6,000 to 20,000 intracardiac electrograms (EGMs) may be captured, which does not lend itself to being manually inspected in full by a clinician (e.g., a physician) for a diagnostic assessment, EGM categorization, and/or the like. Typically mapping systems extract scalar values from each EGM to construct voltage, activation, or other map types to depict overall patterns of activity within the heart. While maps reduce the need to inspect the captured EGMs, they also condense the often complex and useful information in the EGMs. Further, maps may be misleading due to electrical artifacts or inappropriate selection of features such as activation times. Additionally, due to the complex nature of conventional techniques, cardiac maps often are not suitable for accurate and efficient interpretation. US 2018/296108 A1, US 2018/325401 A1, and US 2012/184863 A1 disclose different approaches of accurate interpretation of cardiac maps.

SUMMARY

While multiple embodiments are disclosed, still other embodiments of the present invention will become apparent to those skilled in the art from the following detailed description, which shows and describes illustrative embodiments of the invention. Accordingly, the drawings and detailed description are to be regarded as illustrative in nature and not restrictive. The invention is defined by the appended claims.

BRIEF DESCRIPTION OF THE DRAWINGS

FIG. 1 shows a schematic diagram of an exemplary embodiment of an electrophysiology system.
FIG. 2 is a block diagram of an illustrative processing unit, in accordance with embodiments of the present disclosure.
FIG. 3A is an example flow diagram depicting an illustrative method of processing cardiac electrical signals to assess importance metrics.
FIG. 3B is an example flow diagram depicting an illustrative method of processing cardiac electrical signals to assess importance metrics.
FIG. 4A depicts an illustrative example of a plurality of cardiac electrical signals and a plurality of corresponding activation waveforms.
FIG. 4B depicts an illustrative example of cardiac electrical signals, activation waveforms and normalized average values.

FIG. 4C depicts an illustrative example of an activation zone.
FIG. 4D depicts an illustrative example of activation waveforms, novelty waveforms and novelty scores.
FIG. 4E depicts an illustrative example of the cardiac electrical signals with corresponding importance metrics and cardiac electrical signal selection.
FIG. 4F depicts an illustrative example of a plurality of cardiac electrical signals with selected cardiac electrical signals.

DETAILED DESCRIPTION

As the terms are used herein with respect to measurements (e.g., dimensions, characteristics, attributes, components, etc.), and ranges thereof, of tangible things (e.g., products, inventory, etc.) and/or intangible things (e.g., data, electronic representations of currency, accounts, information, portions of things (e.g., percentages, fractions), calculations, data models, dynamic system models, algorithms, parameters, etc.), "about" and "approximately" may be used, interchangeably, to refer to a measurement that includes the stated measurement and that also includes any measurements that are reasonably close to the stated measurement, but that may differ by a reasonably small amount such as will be understood, and readily ascertained, by individuals having ordinary skill in the relevant arts to be attributable to measurement error; differences in measurement and/or manufacturing equipment calibration; human error in reading and/or setting measurements; adjustments made to optimize performance and/or structural parameters in view of other measurements (e.g., measurements associated with other things); particular implementation scenarios; imprecise adjustment and/or manipulation of things, settings, and/or measurements by a person, a computing device, and/or a machine; system tolerances; control loops; machine-learning; foreseeable variations (e.g., statistically insignificant variations, chaotic variations, system and/or model instabilities, etc.); preferences; and/or the like.

Although illustrative methods may be represented by one or more drawings (e.g., flow diagrams, communication flows, etc.), the drawings should not be interpreted as implying any requirement of, or particular order among or between, various steps disclosed herein. However, some embodiments may require certain steps and/or certain orders between certain steps, as may be explicitly described herein and/or as may be understood from the nature of the steps themselves (e.g., the performance of some steps may depend on the outcome of a previous step). Additionally, a "set," "subset," or "group" of items (e.g., inputs, algorithms, data values, etc.) may include one or more items, and, similarly, a subset or subgroup of items may include one or more items. A "plurality" means more than one.

As used herein, the term "based on" is not meant to be restrictive, but rather indicates that a determination, identification, prediction, calculation, and/or the like, is performed by using, at least, the term following "based on" as an input. For example, predicting an outcome based on a particular piece of information may additionally, or alternatively, base the same determination on another piece of information.

Embodiments of the present disclosure facilitate assessing importance metrics for signals collected from electrodes from the perspectives of contributing to cardiac beats. In embodiments, an importance metric represents a contribution to a duty cycle of a cardiac beat having a cycle length, where a duty cycle represents a percent of activation during the cycle length. Embodiments of the present disclosure facilitate assessing importance metrics of signals collected from electrodes based on activation waveforms generated from the cardiac electrical signals. An activation waveform, or referred to as an annotation waveform, is a set of activation waveform values and may include, for example, a set of discrete activation waveform values (e.g., a set of activation waveform values, a set of activation time annotations, etc.), a function defining an activation waveform curve, and/or the like. In some embodiments, each data point of an activation waveform represents the per-sample "probability" of tissue activation. In some embodiments, the cardiac electrical signals and/or the activation waveform may be displayed, used to present in an activation propagation map, used to facilitate diagnoses, used to facilitate classification (e.g., importance metrics) of cardiac electrical signals, and/or the like. To perform aspects of embodiments of the methods described herein, the cardiac electrical signals may be obtained from a mapping catheter (e.g., associated with a mapping system), which may be used in conjunction with other equipment typically used in an electrophysiology lab, e.g., a recording system, a coronary sinus (CS) catheter or other reference catheter, an ablation catheter, a memory device (e.g., a local memory, a cloud server, etc.), a communication component, a medical device (e.g., an implantable medical device, an external medical device, a telemetry device, etc.), and/or the like.

As the term is used herein, a sensed cardiac electrical signal may refer to one or more sensed signals. Each cardiac electrical signal may include intracardiac electrograms (EGMs) sensed within a patient's heart and may include any number of features that may be ascertained by aspects of an electrophysiology system. A cardiac electric signal, also referred to as an electrical signal, may be an electrogram (EGM), a filtered EGM, a set of absolute values of an EGM, values of peaks of an EGM at peak locations, a combination of these, and/or the like. For example, a cardiac electrical signal may be represented as a set of ordered values (e.g., the amplitude of each sample point may be a value in the set), and a specified percentile and/or multiplier thereof, may be used to define a signal baseline.

Examples of cardiac electrical signal features include, but are not limited to, activation times, activations, activation waveforms, filtered activation waveforms, minimum voltage values, maximum voltages values, maximum negative time-derivatives of voltages, instantaneous potentials, voltage amplitudes, dominant frequencies, peak-to-peak voltages, and/or the like. A cardiac electrical signal feature may refer to one or more features extracted from one or more cardiac electrical signals, derived from one or more features that are extracted from one or more cardiac electrical signals, and/or the like. In embodiments, the cardiac electrical signal feature may include a importance metric. Additionally, a representation of a cardiac electrical signal feature may represent one or more cardiac electrical signal features, an interpolation of a number of cardiac electrical signal features, and/or the like. In some cases, a representation of the cardiac electrical signal is on a cardiac and/or a surface map.

Each cardiac signal also may be associated with a set of respective position coordinates that corresponds to the location at which the cardiac electrical signal was sensed. Each of the respective position coordinates for the sensed cardiac signals may include three-dimensional Cartesian coordinates, polar coordinates, and/or the like. In some cases, other coordinate systems can be used. In some embodiments, an arbitrary origin is used and the respective position coordinates refer to positions in space relative to the arbitrary origin. Since, in some embodiments, the cardiac signals may be sensed on the cardiac surfaces, the respective position coordinates may be on the endocardial surface, epicardial surface, in the mid-myocardium of the patient's heart, and/or in the vicinity of one of one of these.

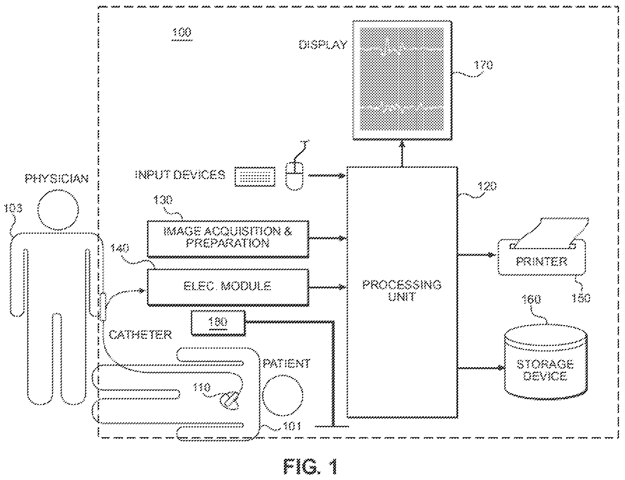

FIG. 1 shows a schematic diagram of an exemplary embodiment of an electrophysiology system 100. As indicated above, embodiments of the subject matter disclosed herein may be implemented in an electrophysiology system (e.g., a mapping system, a cardiac mapping system), while other embodiments may be implemented in an ablation system, a recording system, a computer analysis system, and/or the like. The electrophysiology system 100 includes a moveable catheter 110 having multiple spatially distributed electrodes. During a signal-acquisition stage, the catheter 110 is displaced to multiple locations within the heart chamber into which the catheter 110 is inserted. In some embodiments the distal end of the catheter 110 is fitted with multiple electrodes spread somewhat uniformly over the catheter. For example, the electrodes may be mounted on the catheter 110 following a 3D olive shape, a basket shape, and/or the like. The electrodes are mounted on a device capable of deploying the electrodes into the desired shape while inside the heart, and retracting the electrodes when the catheter is removed from the heart. To allow deployment into a 3D shape in the heart, electrodes may be mounted on a balloon, shape memory material such as Nitinol, actuable hinged structure, and/or the like. According to embodiments, the catheter 110 may be a mapping catheter, an ablation catheter, a diagnostic catheter, a CS catheter, and/or the like. For example, aspects of embodiments of the catheter 110, the electrical signals obtained using the catheter 110, and subsequent processing of the electrical signals, as described herein, may also be applicable in implementations having a recording system, ablation system, and/or any other system having a catheter with electrodes that may be configured to obtain cardiac electrical signals.

At each of the locations to which the catheter 110 is moved, the catheter's multiple electrodes acquire signals resulting from the electrical activity in the heart. Consequently, reconstructing and presenting to a user (such as a doctor and/or technician) physiological data pertaining to the heart's electrical activity may be based on information acquired at multiple locations, thereby providing a more accurate and faithful reconstruction of physiological behavior of the endocardium surface. The acquisition of signals at multiple catheter locations in the heart chamber enables the catheter to effectively act as a "mega-catheter" whose effective number of electrodes and electrode span is proportional to the product of the number of locations in which signal acquisition is performed and the number of electrodes the catheter has.

To enhance the quality of the reconstructed physiological information at the endocardium surface, in some embodiments the catheter 110 is moved to more than three locations (for example, more than 5, 10, or even 50 locations) within the heart chamber. Further, the spatial range over which the catheter is moved may be larger than one third (1/3) of the diameter of the heart cavity (for example, larger than 35%, 40%, 50% or even 60% of the diameter of the heart cavity). Additionally, in some embodiments the reconstructed physiological information is computed based on signals measured over several heart beats, either at a single catheter location within the heart chamber or over several locations. In circumstances where the reconstructed physiological information is based on multiple measurements over several heart beats, the measurements may be synchronized with one another so that the measurements are performed at approximately the same phase of the heart cycle. The signal measurements over multiple beats may be synchronized based on features detected from physiological data such as surface electrocardiograms (ECGs) and/or intracardiac electrograms (EGMs).

The electrophysiology system 100 further includes a processing unit 120 which performs several of the operations pertaining to the mapping procedure, including the reconstruction procedure to determine the physiological information at the endocardium surface (e.g., as described above) and/or within a heart chamber. The processing unit 120 also may perform a catheter registration procedure. The processing unit 120 also may generate a 3D grid used to aggregate the information captured by the catheter 110 and to facilitate display of portions of that information.

The location of the catheter 110 inserted into the heart chamber can be determined using a conventional sensing and tracking system 180 that provides the 3D spatial coordinates of the catheter and/or its multiple electrodes with respect to the catheter's coordinate system as established by the sensing and tracking system. These 3D spatial locations may be used in building the 3D grid. Embodiments of the system 100 may use a hybrid location technology that combines impedance location with magnetic location technology. This combination may enable the system 100 to accurately track catheters that are connected to the system 100. Magnetic location technology uses magnetic fields generated by a localization generator positioned under the patient table to track catheters with magnetic sensors. Impedance location technology may be used to track catheters that may not be equipped with a magnetic location sensor, which may be used with surface ECG patches.

In some embodiments, to perform a mapping procedure and reconstruct physiological information on the endocardium surface, the processing unit 120 may align the coordinate system of the catheter 110 with the endocardium surface's coordinate system. The processing unit 120 (or some other processing component of the system 100) may determine a coordinate system transformation function that transforms the 3D spatial coordinates of the catheter's locations into coordinates expressed in terms of the endocardium surface's coordinate system, and/or vice-versa. In some cases, such a transformation may not be necessary, as some embodiments of the 3D grid may be used to capture contact and non-contact EGMs, and select mapping values based on statistical distributions associated with nodes of the 3D grid. The processing unit 120 also may perform post-processing operations on the physiological information to extract and display useful features of the information to the operator of the system 100 and/or other persons (e.g., a physician).

According to embodiments, the signals acquired by the multiple electrodes of catheter 110 are passed to the processing unit 120 via an electrical module 140, which may include, for example, a signal conditioning component. The electrical module 140 receives the signals communicated from the catheter 110 and performs signal enhancement operations on the signals before they are forwarded to the processing unit 120. The electrical module 140 may include signal conditioning hardware, software, and/or firmware that may be used to amplify, filter and/or sample intracardiac potential measured by one or more electrodes. The intracardiac signals typically have a maximum amplitude of 60mV, with a mean of a few millivolts.

In some embodiments, the signals are filtered by a bandpass filter with a frequency range (e.g., 0.5-500Hz) and sampled with analog to digital converters (e.g., with 15-bit resolution at 1kHz). To avoid interference with electrical equipment in the room, the signals may be filtered to remove the frequency corresponding to the power supply (e.g., 60 Hz). Other types of signal processing operations such as spectral equalization, automatic gain control, etc. may also take place. In some implementations, the intracardiac signals may be unipolar signals measured relative to a reference (which may be a virtual reference). In such implementations, the reference can be, for example, a coronary sinus catheter or Wilson's Central Terminal (WCT), from which the signal processing operations may compute differences to generate multipolar signals (e.g., bipolar signals, tripolar signals, etc.). In some other implementations, the signals may be processed (e.g., filtered, sampled, etc.) before and/or after generating the multipolar signals. The resultant processed signals are forwarded by the electrical module 140 to the processing unit 120 for further processing.

As further shown in FIG. 1, the electrophysiology system 100 also may include peripheral devices such as a printer 150 and/or display device 170, both of which may be interconnected to the processing unit 120. Additionally, the electrophysiology system 100 includes storage device 160 that may be used to store data acquired by the various interconnected modules, including the volumetric images, raw data measured by electrodes and/or the resultant endocardium representation computed therefrom, the partially computed transformations used to expedite the mapping procedures, the reconstructed physiological information corresponding to the endocardium surface, and/or the like.

In some embodiments, the processing unit 120 may be configured to automatically improve the accuracy of its algorithms by using one or more artificial intelligence techniques (e.g., machine learning models, deep learning models), classifiers, and/or the like. In some embodiments, for example, the processing unit may use one or more supervised and/or unsupervised techniques such as, for example, support vector machines (SVMs), k-nearest neighbor techniques, neural networks, convolutional neural networks, recurrent neural networks, and/or the like. In some embodiments, classifiers may be trained and/or adapted using feedback information from a user, other metrics, and/or the like.

The illustrative electrophysiology system 100 shown in FIG. 1 is not intended to suggest any limitation as to the scope of use or functionality of embodiments of the present disclosure. Neither should the illustrative electrophysiology system 100 be interpreted as having any dependency or requirement related to any single component or combination of components illustrated therein. Additionally, various components depicted in FIG. 1 may be, in some embodiments, integrated with various ones of the other components depicted therein (and/or components not illustrated), all of which are considered to be within the ambit of the subject matter disclosed herein. For example, the electrical module 140 may be integrated with the processing unit 120. Additionally, or alternatively, aspects of embodiments of the electrophysiology system 100 may be implemented in a computer analysis system configured to receive cardiac electrical signals and/or other information from a memory device (e.g., a cloud server, a mapping system memory, etc.), and perform aspects of embodiments of the methods described herein for processing cardiac information (e.g., determining annotation waveforms, etc.). That is, for example, a computer analysis system may include a processing unit 120, but not a mapping catheter.

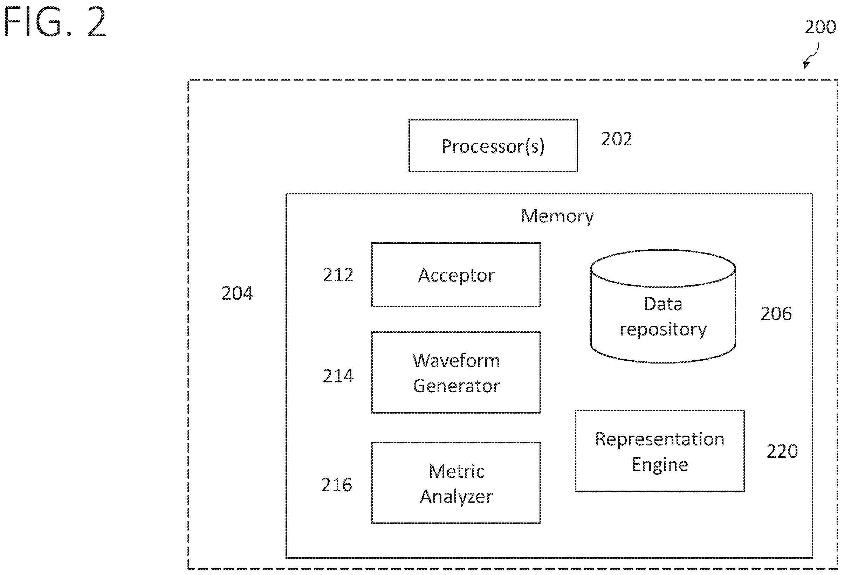

FIG. 2 is a block diagram of an illustrative processing unit 200, in accordance with embodiments of the present disclosure. The processing unit 200 may be, be similar to, include, or be included in the processing unit 120 depicted in FIG. 1. As shown in FIG. 2, the processing unit 200 may be implemented on a computing device that includes one or more processors 202 and one or more memories 204. Although the processing unit 200 is referred to herein in the singular, the processing unit 200 may be implemented in multiple instances (e.g., as a server cluster), distributed across multiple computing devices, instantiated within multiple virtual machines, and/or the like. One or more components for facilitating cardiac mapping may be stored in the memory 204. In some embodiments, the processor 202 may be configured to instantiate the one or more components to generate an activation waveform, a set of metric analysis results, a set of waveform analysis results, electrogram characteristics, a histogram, and a cardiac map, any one or more of which may be stored in the data repository 206.

As depicted in FIG. 2, the processing unit 200 may include an acceptor 212 configured to receive electrical signals from a mapping catheter (e.g., the mapping catheter 110 depicted in FIG. 1). The measured electrical signals may include a number of intracardiac electrograms (EGMs) sensed within a patient's heart. The acceptor 212 may also receive an indication of a measurement location corresponding to each of the electrical signals. In some embodiments, the acceptor 212 may be configured to determine whether to accept the electrical signals that have been received. The acceptor 212 may utilize any number of different components and/or techniques to determine which electrical signals or beats to accept, such as filtering, beat matching, morphology analysis, positional information (e.g., catheter motion), respiration gating, and/or the like. The received electrical signals and/or the processed electrical signals may be stored in the data repository 206.

The accepted electrical signals are received by an activation waveform generator 214 that is configured to extract at least one annotation feature from each of the electrical signals, in cases in which the electrical signal includes an annotation feature to extract. In some embodiments, the at least one annotation feature includes at least one value corresponding to at least one activation metric. The at least one feature may include at least one event, where the at least one event includes the at least one value corresponding to the at least one metric and/or at least one corresponding time (a corresponding time does not necessarily exist for each activation feature). In some embodiments, the at least one metric may include, for example, an activation time, minimum voltage value, maximum voltage value, maximum negative time-derivative of voltage, an instantaneous potential, a voltage amplitude, a dominant frequency, a peak-to-peak voltage, an activation duration, and/or the like. In some embodiments, the activation waveform generator 214 may be configured to detect activations and to generate an activation waveform. In some cases, the waveform generator 214 can use any one of activation waveform embodiments, for example, including those described in U.S. Patent Publication 2018/0296113, entitled "ANNOTATION WAVEFORM," the disclosure of which is hereby expressly incorporated herein by reference.

As illustrated in FIG. 2, the processing unit 200 includes a metric analyzer 216 to analyze the received cardiac electrical signals and/or the activation waveform generated by the activation waveform generator 214 to determine metrics associated with the cardiac electrical signals. In embodiments, the metric analyzer 216 is configured to determine importance metrics and other metrics of the cardiac electrical signals. In some embodiments, the metric analyzer 216 is configured to determine whether a cardiac electrical signal meaningfully contribute a cardiac beat by evaluating the activation waveform corresponding to the signal. Additionally, the processing unit 200 includes a representation engine 220 that is configured to facilitate presentation of a representation of cardiac electrical signals and/or the metric analysis results, for example, selected cardiac electrical signals having important metrics equal to or greater than a predetermined threshold.

The illustrative processing unit 200 shown in FIG. 2 is not intended to suggest any limitation as to the scope of use or functionality of embodiments of the present disclosure. Neither should the illustrative processing unit 200 be interpreted as having any dependency or requirement related to any single component or combination of components illustrated therein. Additionally, any one or more of the components depicted in FIG. 2 may be, in some embodiments, integrated with various ones of the other components depicted therein (and/or components not illustrated), all of which are considered to be within the ambit of the subject matter disclosed herein. For example, the waveform generator 214 may be integrated with the metric analyzer 216. In some embodiments, the processing unit 200 may not include an acceptor 212, while in other embodiments, the acceptor 212 may be configured to receive electrical signals from a memory device, a communication component, and/or the like.

Additionally, the processing unit 200 may (alone and/or in combination with other components of the system 100 depicted in FIG. 1, and/or other components not illustrated) perform any number of different functions and/or processes associated with cardiac mapping (e.g., triggering, blanking, field mapping, etc.) such as, for example, those described in U.S. Patent Publication 2018/0296113, entitled "ANNOTATION WAVEFORM;" U.S. Patent 8,428,700, entitled "ELECTROANATOMICAL MAPPING;" U.S. Patent 8,948,837, entitled "ELECTROANATOMICAL MAPPING;" U.S. Patent 8,615,287, entitled "CATHETER TRACKING AND ENDOCARDIUM REPRESENTATION GENERATION;" U.S. Patent Publication 2015/0065836, entitled "ESTIMATING THE PREVALENCE OF ACTIVATION PATTERNS IN DATA SEGMENTS DURING ELECTROPHYSIOLOGY MAPPING;" U.S. Patent 6,070,094, entitled "SYSTEMS AND METHODS FOR GUIDING MOVABLE ELECTRODE ELEMENTS WITHIN MULTIPLE-ELECTRODE STRUCTURE;" U.S. Patent 6,233,491, entitled "CARDIAC MAPPING AND ABLATION SYSTEMS," U.S. Patent 6,735,465, entitled "SYSTEMS AND PROCESSES FOR REFINING A REGISTERED MAP OF A BODY CAVITY;" the disclosures of which are hereby expressly incorporated herein by reference.

According to embodiments, various components of the electrophysiology system 100, illustrated in FIG. 1, and/or the processing unit 200, illustrated in FIG. 2, may be implemented on one or more computing devices. A computing device may include any type of computing device suitable for implementing embodiments of the disclosure. Examples of computing devices include specialized computing devices or general-purpose computing devices such "workstations," "servers," "laptops," "desktops," "tablet computers," "hand-held devices," "general-purpose graphics processing units (GPGPUSs)," and the like, all of which are contemplated within the scope of FIGS. 1 and 2 with reference to various components of the system 100 and/or processing unit 200.

In some embodiments, a computing device includes a bus that, directly and/or indirectly, couples the following devices: a processor, a memory, an input/output (I/O) port, an I/O component, and a power supply. Any number of additional components, different components, and/or combinations of components may also be included in the computing device. The bus represents what may be one or more busses (such as, for example, an address bus, data bus, or combination thereof). Similarly, in some embodiments, the computing device may include a number of processors, a number of memory components, a number of I/O ports, a number of I/O components, and/or a number of power supplies. Additionally, any number of these components, or combinations thereof, may be distributed and/or duplicated across a number of computing devices.

In some embodiments, memory (e.g., the storage device 160 depicted in FIG. 1, the memory 204 and/or the data repository 206 depicted in FIG. 2) includes computer-readable media in the form of volatile and/or nonvolatile memory, transitory and/or non-transitory storage media and may be removable, nonremovable, or a combination thereof. Media examples include Random Access Memory (RAM); Read Only Memory (ROM); Electronically Erasable Programmable Read Only Memory (EEPROM); flash memory; optical or holographic media; magnetic cassettes, magnetic tape, magnetic disk storage or other magnetic storage devices; data transmissions; and/or any other medium that can be used to store information and can be accessed by a computing device such as, for example, quantum state memory, and/or the like. In some embodiments, the memory 160 and/or 204 stores computer-executable instructions for causing a processor (e.g., the processing unit 120 depicted in FIG. 1 and/or the processor 202 depicted in FIG. 2) to implement aspects of embodiments of system components discussed herein and/or to perform aspects of embodiments of methods and procedures discussed herein.

Computer-executable instructions may include, for example, computer code, machine-useable instructions, and the like such as, for example, program components capable of being executed by one or more processors associated with a computing device. Examples of such program components include the acceptor 212, the waveform generator 214, the metric analyzer 216, and the representation engine 220. Program components may be programmed using any number of different programming environments, including various languages, development kits, frameworks, and/or the like. Some or all of the functionality contemplated herein may also, or alternatively, be implemented in hardware and/or firmware.

The data repository 206 may be implemented using any one of the configurations described below. A data repository may include random access memories, flat files, XML files, and/or one or more database management systems (DBMS) executing on one or more database servers or a data center. A database management system may be a relational (RDBMS), hierarchical (HDBMS), multidimensional (MDBMS), object oriented (ODBMS or OODBMS) or object relational (ORDBMS) database management system, and the like. The data repository may be, for example, a single relational database. In some cases, the data repository may include a plurality of databases that can exchange and aggregate data by data integration process or software application. In an exemplary embodiment, at least part of the data repository 206 may be hosted in a cloud data center. In some cases, a data repository may be hosted on a single computer, a server, a storage device, a cloud server, or the like. In some other cases, a data repository may be hosted on a series of networked computers, servers, or devices. In some cases, a data repository may be hosted on tiers of data storage devices including local, regional, and central.

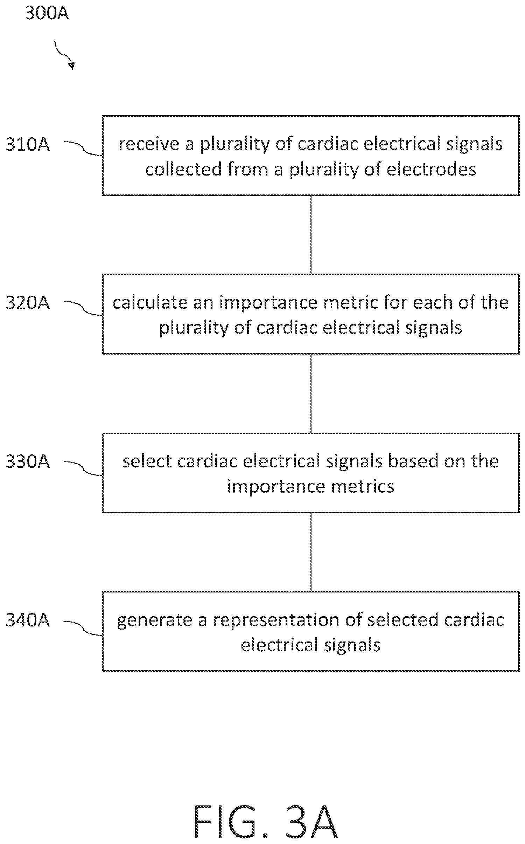

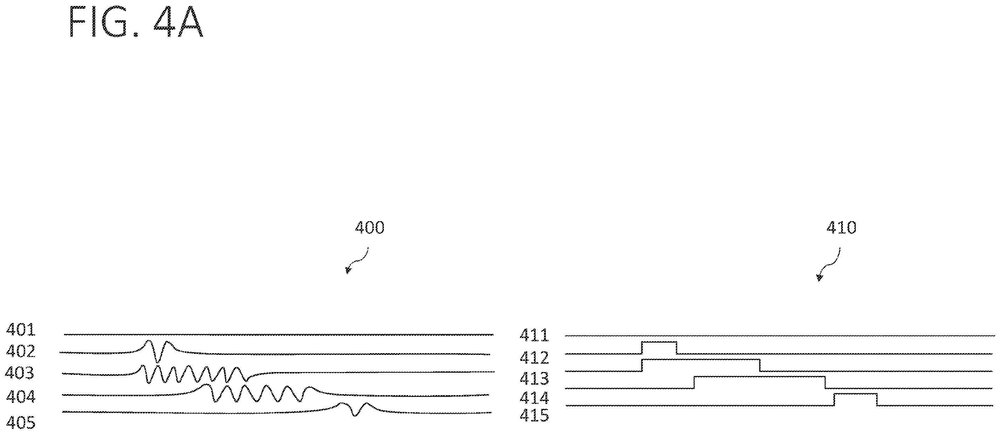

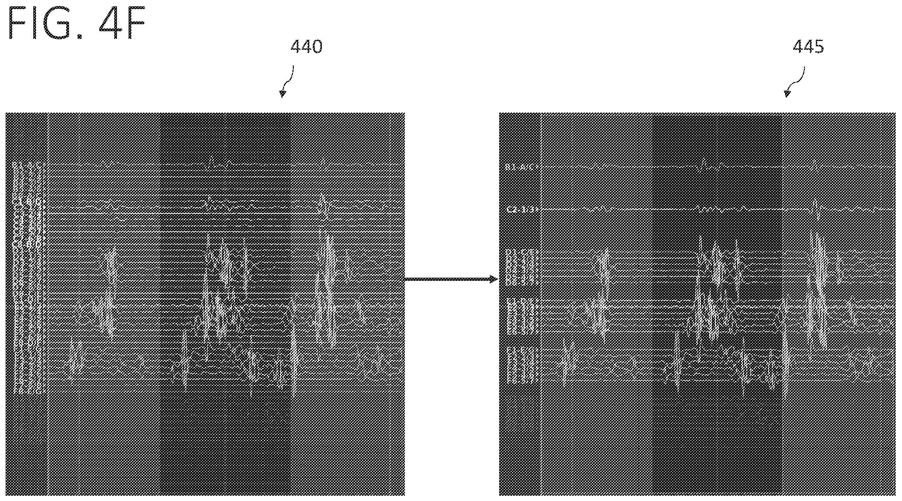

FIG. 3A is an example flow diagram depicting an illustrative method 300A of processing cardiac electrical signals to assess importance metrics, in accordance with some embodiments of the present disclosure. Aspects of embodiments of the method 300A may be performed, for example, by an electrophysiology system or a processing unit (e.g., the processing unit 120 depicted in FIG. 1, and/or the processing unit 200 depicted in FIG. 2). One or more steps of method 300A are optional and/or can be modified by one or more steps of other embodiments described herein. Additionally, one or more steps of other embodiments described herein may be added to the method 300A. First, the electrophysiology system receives a plurality of cardiac electrical signals collected from a plurality of electrodes disposed within a cardiac chamber (310A), where the plurality of cardiac electrical signals are acquired over a cardiac beat having a cycle length. FIG. 4A depicts an illustrative example of a plurality of cardiac electrical signals (e.g., EGMs) over a cardiac beat. FIG. 4F depicts an illustrative example of a plurality of cardiac electrical signals 440 over a few cardiac beats.

The system calculates an importance metric for each of the plurality of cardiac electrical signals (320A). In embodiments, the importance metric represents a contribution of a respective cardiac electrical signal to an overall duty cycle of the cardiac beat as a function of the cycle length. In some cases, the contribution to an overall duty cycle includes a contribution to the main activation period of a cardiac beat, also referred to as an activation metric. In some cases, the main activation period of a cardiac beat is determined based on one or more cardiac electrical signals that are main contributors to the activation of the cardiac beat (e.g., signals contributing to more than 60% of the beat's duty cycle). In embodiments, the contribution to an overall duty cycle includes a contribution outside the main activation period of the cardiac beat, also referred to as a novelty metric. The novelty metric can represent the uniqueness of signals collected by one or more electrodes.

The electrophysiology system may select cardiac electrical signals based on the importance metrics (330A). In some embodiments, the system can select cardiac electrical signals based on one or more criteria, where at least one of the criteria uses the importance metric as a parameter. In one embodiment, at least one of the criteria is a respective importance metric equal to or greater than a predetermined threshold. In one embodiment, at least one of the criteria aggregates the importance metric with at least one other characteristics of the cardiac electric signal. The system can generate a representation of selected cardiac electrical signals (340A). In one embodiment, the representation is a graphical representation. FIG. 4F depicts an illustrative example of a graphical representation of selected cardiac electrical signals 445.

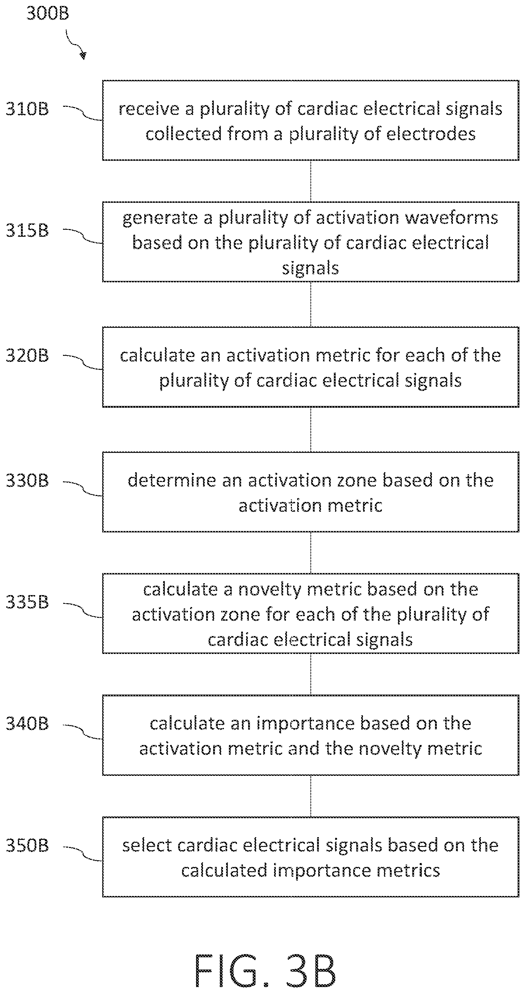

FIG. 3B is an example flow diagram depicting an illustrative method 300B of processing cardiac electrical signals to assess importance metrics, in accordance with some embodiments of the present disclosure. Aspects of embodiments of the method 300B may be performed, for example, by an electrophysiology system or a processing unit (e.g., the processing unit 120 depicted in FIG. 1, and/or the processing unit 200 depicted in FIG. 2). One or more steps of method 300B are optional and/or can be modified by one or more steps of other embodiments described herein. Additionally, one or more steps of other embodiments described herein may be added to the method 300B. First, the electrophysiology system receives a plurality of cardiac electrical signals collected from a plurality of electrodes (310B), over a cardiac beat. The system may generate a plurality of activation waveforms corresponding to the plurality of cardiac electrical signals (315B).

In one embodiment, the electrophysiology system identifies a deflection for each of the plurality of electrical signals, where the deflection is a deviation from a signal baseline. Each of the plurality of activation waveforms is generated based on one or more identified deflections of an electrical signal. The activation waveform includes values corresponding to probabilities that the identified deflections represent activations of cardiac tissue. The system may also calculate an activation metric for each of the cardiac electrical signals (320B). In some cases, the activation metric is calculated based on the corresponding activation waveform. In one example, the activation metric can be calculated based on the activation waveform values. In some embodiments, the activation metric represents a contribution of a cardiac electrical signal within an activation zone that is explained in more detail below. FIG. 4A depicts an illustrative example of a plurality of cardiac electrical signals 400 (e.g., signals 401, 402, 403, 404 and 405) and a plurality of corresponding activation waveforms 410 (e.g., waveforms 411, 412, 413, 414 and 415) each for a respective cardiac electrical signal. In one implementation, an arithmetic average value of the activation values of the activation waveform is calculated. In the example illustrated in FIG. 4A, the arithmetic average values for the activation waveforms 411, 412, 413, 414, 415 are 0, 0.05, 0.20, 0.25, and 0.08 respectively.

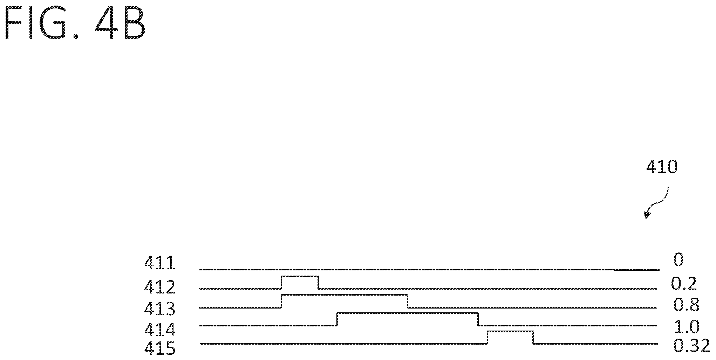

In one implementation, the system further normalizes the arithmetic average values, where the normalized values are referred to as normalized average values, of the activation waveforms of the plurality cardiac electrical signals. In one example, the largest arithmetic average value is set to 1 by a multiplier and the other arithmetic average values are normalized using the same multiplier. FIG. 4B depicts an illustrative example of activation waveforms 410 and normalized average values. In this example, the normalized average values of the activation waveforms 411, 412, 413, 414, 415 are 0, 0.20, 0.80, 1.00, and 0.32 respectively. In some embodiments, the activation metric of a cardiac electrical signal is set to the normalized average value of the corresponding activation waveform.

Referring back to FIG. 3B, the electrophysiology system may determine an activation zone (330B), based on the plurality of activation waveforms corresponding to the plurality of cardiac electrical signals. In one embodiment, the system selects one or more activation waveforms based on the normalized average values. For example, an activation waveform is selected if its normalized average value is greater than a predetermined mean-value threshold. In some cases, the predetermined mean-value threshold is 0.6. Next, the system determines an activation zone based on the selected activation waveforms. In one example, the activation zone is represented by determined using equation (1) below:

$$AZ(t) = max(\forall\, AW_S(t)|Metric_A > 0.6) \qquad (1),$$

where $AZ(t)$ is the activation zone as a function of time, $AW_S(t)$ is the activation waveforms of the selected electrical signals, and $Metric_A$ is the activation metric of an electrical signal.

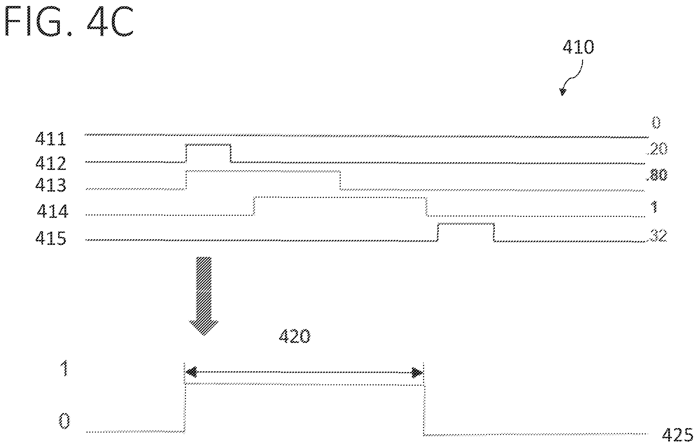

FIG. 4C depicts an illustrative example of an activation zone. In the example illustrated in FIG. 4C, the system selects activation waveforms 413 and 414 corresponding to cardiac electrical signals 403 and 404 with a predetermined mean-value threshold of 0.6 and use the selected activation waveforms 413 and 414 to determine an activation zone 420 represented by a waveform 425.

In some embodiments, the system calculates a novelty metric for each of the plurality of cardiac electrical signals (335B). In some embodiments, the novelty metric is calculated based on each activation waveform outside of the activation zone. In some embodiments, the activation zone is used as inversed weight factors (i.e., higher activation values corresponding to lower weight factors) to determine the novelty metric. In one example, a novelty waveform is generated using equation (2) below:

$$NW_{CS}(t) = (1 - AZ(t)) \times AW_{CS}(t) \qquad (2),$$

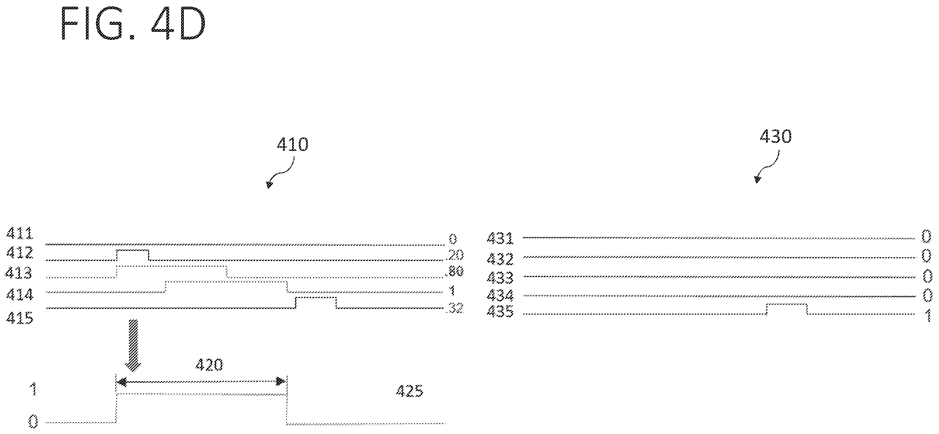

where $NW_{CS}(t)$ is the novelty waveform of a cardiac electrical signal as a function of time, $AZ(t)$ is the activation zone as a function of time, and $AW_{CS}(t)$ is the activation waveform of the cardiac electrical signal. In one embodiment, a novelty metric is determined based on the novelty waveform. In one case, the novelty metric is set to the maximum value of the novelty waveform. FIG. 4D depicts an illustrative example of activation waveforms 410, novelty waveforms 430 and novelty scores. As illustrated, the novelty waveforms 431, 432, 433, 434 and 435 are generated based on the activation waveforms 411, 412, 413, 414 and 415 and the activation zone waveform 425. In the example of FIG. 4D, the novelty metrics of the cardiac electrical signals corresponding to the activation waveforms 411, 412, 413, 414 and 415 are 0, 0, 0, 0 and 1 respectively.

In some embodiments, the electrophysiology system calculates an importance metric for each of the plurality of cardiac electrical signals based on the corresponding activation metric and novelty metric (340B). In one embodiment, the importance metric is determined using equation (3) below:

$$Metric_I = f(Metric_A, Metric_N) \qquad (3),$$

where $Metric_I$ is the importance metric, $Metric_A$ is the activation metric, $Metric_N$ is the novelty metric, and $f()$ is a function. In some cases, the function $f()$ is a linear function. In some cases, the function $f()$ is a non-linear function. In some cases, the function $f()$ is an error function. In one example, the importance metric is determined using equation (4) below:

$$Metric_I = \frac{\mathrm{erf}(6 \times (max(Metric_A, Metric_N) - 0.5)) + 1}{2} \qquad (4),$$

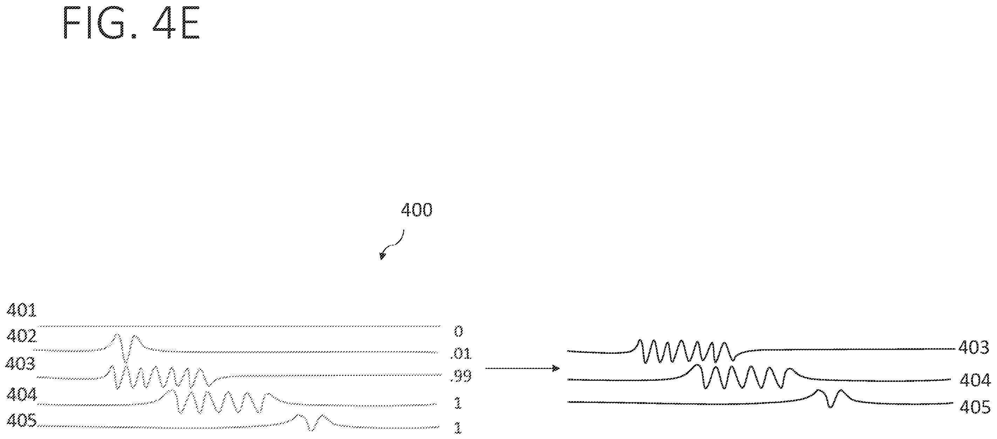

where $Metric_I$ is the importance metric, $Metric_A$ is the activation metric, $Metric_N$ is the novelty metric, and $erf()$ is an error function. FIG. 4E depicts an illustrative example of the cardiac electrical signals 400 with corresponding importance metrics and cardiac electrical signal selection. In the example, the cardiac electrical signals 401, 402, 403, 404 and 405 have importance metrics 0, 0.01, 0,99, 1, and 1 respectively.

In some embodiments, the system selects the cardiac electrical signals based on the calculated importance metric (350B). In one example, a cardiac electrical signal is selected if its calculated importance metric is greater than a predetermined threshold. FIG. 4E depicts an illustrative example of the cardiac electrical signals 401, 402, 403, 404 and 405 and the cardiac electrical signals 403, 404, and 405 being selected based on the respective importance metrics. In some cases, a cardiac electrical signal is selected if a set of criteria is met. In some designs, a criteria uses the importance metric as a parameter. For example, the set of criteria includes a criteria of a calculated importance metric greater than a predetermined threshold. FIG. 4F depicts an illustrative example of a plurality of cardiac electrical signals 440 with selected cardiac electrical signals 445, where the signal selection and the selection of corresponding electrodes (e.g., unipolar electrodes, bipolar electrodes, tripolar electrodes, etc.) are based on a criteria using importance metric as a parameter.

## Claims

1. A system for processing cardiac information, the system comprising:
   a processing unit configured to:

      receive a plurality of cardiac electrical signals (403, 404) collected from a plurality of electrodes disposed within a cardiac chamber, wherein the plurality of cardiac electrical signals are acquired over a cardiac beat having a cycle length;
      generate a plurality of activation waveforms (413, 414) based on the plurality of cardiac electrical signals;
      determine an activation zone (420) based on the activation waveforms;
      calculate an importance metric for each of the plurality of the cardiac electrical signals, wherein the importance metric represents a contribution of a respective cardiac electrical signal to an overall duty cycle of the cardiac beat as a function of the cycle length, wherein the importance metric is calculated based on an activation metric and a novelty metric, wherein the activation metric represents a first contribution of the respective cardiac electrical signal to the activation zone, and wherein the novelty metric represents a second contribution of the respective cardiac electrical signal to outside the activation zone; and

facilitate presentation, on a display device, a graphical representation of selected cardiac electrical signals, wherein each of the selected cardiac electrical signals meets a selection criteria based on a respective importance metric.

2. The system of claim 1, wherein each of the plurality of cardiac electrical signals includes an intra-cardiac electrogram (EGM).

3. The system of claim 1, wherein the processing unit is further configured to:

identify, for each of the plurality of electrical signals, a deflection, the deflection comprising a deviation from a signal baseline,
wherein each of the plurality of activation waveforms is generated based on a respective identified deflection, wherein the activation waveform comprises activation waveform values corresponding to probabilities that the identified deflections represent activations of cardiac tissue.

4. The system of claim 3, wherein the activation metric is calculated based on an average value of the activation waveform values.

5. The system of claim 4, wherein the activation metric is calculated by normalizing the average value of the activation waveform values.

6. The system of claim 1, further comprising:

selecting one or more activation waveforms based on the activation metrics; and
determining an activation zone waveform based on the selected one or more activation waveforms, the activation zone waveform representing the activation zone.

7. The system of claim 6, wherein each of the selected one or more activation waveforms has an activation metric greater than a predetermined activation metric threshold.

8. The system of claim 6, wherein the novelty metric is calculated using weight factors based on the activation zone waveform, wherein a first weight factor is corresponding to a first activation zone value and a second weight factor is corresponding to a second activation zone value, and wherein the first weight factor is greater than the second weight factor with the first activation zone value smaller than the second activation zone value.

9. The system of claim 1, wherein the importance metric is determined by applying a non-linear function to the activation metric and the novelty metric.

10. The system of any one of claims 1-9, wherein the selection criteria comprises the respective importance metric greater than a predetermined threshold.

11. A method of processing cardiac information, the method comprising:

receiving a plurality of cardiac electrical signals collected from a plurality of electrodes disposed within a cardiac chamber, wherein the plurality of cardiac electrical signals are acquired over a cardiac beat having a cycle length;
generating a plurality of activation waveforms based on the plurality of cardiac electrical signals, wherein each of the plurality of activation waveforms is generated based on deflections of the plurality of cardiac electrical signals from a signal baseline, wherein the activation waveform comprises activation waveform values corresponding to probabilities that the identified deflections represent activations of cardiac tissue;
calculating an importance metric for each of the plurality of the cardiac electrical signals, wherein the importance metric represents a contribution of a respective cardiac electrical signal to an overall duty cycle of the cardiac beat as a function of the cycle length, wherein the importance metric is calculated based on an activation metric and a novelty metric, wherein the activation metric represents a first contribution of the respective cardiac electrical signal to an activation zone, wherein the novelty metric represents a second contribution of the respective cardiac electrical signal to outside the activation zone, and wherein the activation metric and the activation zone are determined based on the activation waveform values; and
facilitating presentation, on a display device, a graphical representation of selected cardiac electrical signals, wherein each of the selected cardiac electrical signals meets a selection criteria based on a respective importance metric.

**Patentansprüche**

1. System zur Verarbeitung von kardiologischen Informationen, wobei das System umfasst:
eine Verarbeitungseinheit, die konfiguriert ist zum:

Empfangen einer Vielzahl von kardialen elektrischen Signalen (403, 404), die von einer Vielzahl von Elektroden, die innerhalb einer Herzkammer angeordnet sind, erfasst werden, wobei die Vielzahl von kardialen elektrischen Signalen über einen Herzschlag mit einer Zykluslänge erfasst wird;
Erzeugen einer Vielzahl von Aktivierungswellenformen (413, 414) auf Grundlage der Vielzahl von kardialen elektrischen Signalen;
Bestimmen einer Aktivierungszone (420) auf Grundlage der Aktivierungswellenformen;
Berechnen einer Wichtigkeitsmetrik für jedes der Vielzahl von kardialen elektrischen Signalen, wobei die Wichtigkeitsmetrik einen Beitrag eines jeweiligen elektrischen Herzsignals zu einem Gesamt-Arbeitszyklus des Herzschlags als Funktion der Zykluslänge darstellt, wobei die Wichtigkeitsmetrik auf der Grundlage einer Aktivierungsmetrik und einer Neuheitsmetrik berechnet wird, wobei die Aktivierungsmetrik einen ersten Beitrag des jeweiligen elektrischen Herzsignals zur Aktivierungszone darstellt, und wobei die Neuheitsmetrik einen zweiten Beitrag des jeweiligen elektrischen Herzsignals außerhalb der Aktivierungszone darstellt; und
Ermöglichen der Darstellung, auf einer Anzeigevorrichtung, einer grafischen Darstellung ausgewählter kardialer elektrischer Signale, wobei jedes der ausgewählten kardialen elektrischen Signalen ein Auswahlkriterium erfüllt, das auf einer jeweiligen Wichtigkeitsmetrik basiert.

2. System nach Anspruch 1, wobei jedes von der Vielzahl von kardialen elektrischen Signalen ein intrakardiales Elektrogramm (EGM) umfasst.

3. System nach Anspruch 1, wobei die Verarbeitungseinheit ferner eingerichtet ist, zum:

Identifizieren einer Deflektion für jedes der Vielzahl von elektrischen Signalen, wobei die Deflektion eine Abweichung von einer Signalgrundlinie umfasst,
wobei jede der Vielzahl von Aktivierungswellenformen auf Grundlage einer jeweils identifizierten Deflektion erzeugt wird, wobei die Aktivierungswellenform Aktivierungswellenformwerte umfasst, die Wahrscheinlichkeiten entsprechen, dass die identifizierten Deflektionen Aktivierungen von Herzgewebe darstellen.

4. System nach Anspruch 3, wobei die Aktivierungsmetrik auf Grundlage eines Durchschnittswerts der Aktivierungswellenformwerte berechnet wird.

5. System nach Anspruch 4, wobei die Aktivierungsmetrik durch Normalisierung des Durchschnittswerts der Aktivierungswellenformwerte berechnet wird.

6. System nach Anspruch 1, das ferner umfasst:

das Auswählen einer oder mehrerer Aktivierungswellenformen auf Grundlage der Aktivierungsmetriken; und
das Bestimmen einer Aktivierungszonen-Wellenform auf Grundlage der ausgewählten einen oder mehreren Aktivierungswellenformen, wobei die Aktivierungszonen-Wellenform die Aktivierungszone darstellt.

7. System nach Anspruch 6, wobei jede der ausgewählten einen oder mehreren Aktivierungswellenformen eine Aktivierungsmetrik aufweist, die größer ist als ein vorbestimmter Aktivierungsmetrikschwellenwert.

8. System nach Anspruch 6, wobei die Neuheitsmetrik unter Verwendung von Gewichtungsfaktoren berechnet wird, die auf der Aktivierungszonen-Wellenform basieren, wobei ein erster Gewichtungsfaktor einem ersten Aktivierungszonenwert entspricht und ein zweiter Gewichtungsfaktor einem zweiten Aktivierungszonenwert entspricht, und wobei der erste Gewichtungsfaktor größer ist als der zweite Gewichtungsfaktor, wobei der erste Aktivierungszonenwert kleiner ist als der zweite Aktivierungszonenwert.

9. System nach Anspruch 1, wobei die Wichtigkeitsmetrik durch Anwenden einer nichtlinearen Funktion auf die Aktivierungsmetrik und die Neuheitsmetrik bestimmt wird.

10. System nach einem der Ansprüche 1-9, wobei die Auswahlkriterien die jeweilige Wichtigkeitsmetrik umfassen, die größer als ein vorbestimmter Schwellenwert ist.

11. Verfahren zur Verarbeitung von kardiologischen Informationen, wobei das Verfahren umfasst:

das Empfangen einer Vielzahl von kardialen elektrischen Signalen, die von einer Vielzahl von Elektroden erfasst werden, die innerhalb einer Herzkammer angeordnet sind, wobei die Vielzahl von kardialen elektrischen Signalen über einen Herzschlag mit einer Zykluslänge erfasst wird;

das Erzeugen einer Vielzahl von Aktivierungswellenformen auf Grundlage der Vielzahl von kardialen elektrischen Signalen, wobei jede der Vielzahl von Aktivierungswellenformen auf Grundlage von Deflektionen der Vielzahl von kardialen elektrischen Signalen von einer Signalgrundlinie erzeugt wird, wobei die Aktivierungswellenform Aktivierungswellenformwerte umfasst, die Wahrscheinlichkeiten entsprechen, die die identifizierten Deflektionen Aktivierungen von Herzgewebe darstellen;

das Berechnen einer Wichtigkeitsmetrik für jedes der Vielzahl von kardialen elektrischen Signalen, wobei die Wichtigkeitsmetrik einen Beitrag eines jeweiligen kardialen elektrischen Signals zu einem Gesamt-Arbeitszyklus des Herzschlags als Funktion der Zykluslänge darstellt, wobei die Wichtigkeitsmetrik auf der Grundlage einer Aktivierungsmetrik und einer Neuheitsmetrik berechnet wird, wobei die Aktivierungsmetrik einen ersten Beitrag des jeweiligen kardialen elektrischen Signals zur Aktivierungszone darstellt, und wobei die Neuheitsmetrik einen zweiten Beitrag des jeweiligen kardialen elektrischen Signals außerhalb der Aktivierungszone darstellt; und

das Ermöglichen der Darstellung, auf einer Anzeigevorrichtung, einer grafischen Darstellung ausgewählter kardialer elektrischer Signale, wobei jedes der ausgewählten kardialen elektrischen Signalen ein Auswahlkriterium erfüllt, das auf einer jeweiligen Wichtigkeitsmetrik basiert.

**Revendications**

1. Système pour traiter d'information cardiaque, le système comprenant :
une unité de traitement configurée pour :

recevoir une pluralité de signaux électriques cardiaques (403, 404) collectés à partir d'une pluralité d'électrodes disposées à l'intérieur d'une chambre cardiaque, dans lequel les signaux de la pluralité de signaux électriques cardiaques sont acquis sur un battement cardiaque présentant une longueur de cycle ;

générer une pluralité de formes d'onde d'activation (413, 414) sur la base de la pluralité de signaux électriques cardiaques ;

déterminer une zone d'activation (420) sur la base des formes d'onde d'activation ;

calculer un paramètre d'importance pour chacun de la pluralité des signaux électriques cardiaques, dans lequel le paramètre d'importance représente une contribution d'un signal électrique cardiaque respectif à un cycle opératoire global du battement cardiaque en fonction de la longueur de cycle, dans lequel le paramètre d'importance est calculé sur la base d'un paramètre d'activation et d'un paramètre de nouveauté, dans lequel le paramètre d'activation représente une première contribution du signal électrique cardiaque respectif à la zone d'activation, et dans lequel le paramètre de nouveauté représente une seconde contribution du signal électrique cardiaque respectif à l'extérieur de la zone d'activation ; et

faciliter la présentation, sur un dispositif d'affichage, d'une représentation graphique de signaux électriques cardiaques sélectionnés, dans lequel chacun des signaux électriques cardiaques sélectionnés satisfait un critère de sélection sur la base d'un paramètre d'importance respectif.

2. Système selon la revendication 1, dans lequel chacun de la pluralité de signaux électriques cardiaques inclut un électrogramme intracardiaque (EGM).

3. Système selon la revendication 1, dans lequel l'unité de traitement est en outre configurée pour :

identifier, pour chacun de la pluralité de signaux électriques, une déflexion, la déflexion comprenant une déviation par rapport à une ligne de base de signal,

dans lequel chacune de la pluralité de formes d'onde d'activation est générée sur la base d'une déflexion identifiée respective, et dans lequel la forme d'onde d'activation comprend des valeurs de forme d'onde d'activation correspondant à des probabilités que les déflexions identifiées représentent des activations de tissu cardiaque.

**4.** Système selon la revendication 3, dans lequel le paramètre d'activation est calculé sur la base d'une valeur moyenne des valeurs de forme d'onde d'activation.

**5.** Système selon la revendication 4, dans lequel le paramètre d'activation est calculé en normalisant la valeur moyenne des valeurs de forme d'onde d'activation.

**6.** Système selon la revendication 1, comprenant en outre :

la sélection d'une ou de plusieurs formes d'onde d'activation sur la base des paramètres d'activation ; et
la détermination d'une forme d'onde de zone d'activation sur la base des une ou plusieurs formes d'onde d'activation sélectionnées, la forme d'onde de zone d'activation représentant la zone d'activation.

**7.** Système selon la revendication 6, dans lequel chacune des une ou plusieurs formes d'onde d'activation sélectionnées présente un paramètre d'activation plus grand qu'un seuil de paramètre d'activation prédéterminé.

**8.** Système selon la revendication 6, dans lequel le paramètre de nouveauté est calculé en utilisant des facteurs de pondération sur la base de la forme d'onde de zone d'activation, dans lequel un premier facteur de pondération correspond à une première valeur de zone d'activation et un second facteur de pondération correspond à une seconde valeur de zone d'activation, et dans lequel le premier facteur de pondération est plus grand que le second facteur de pondération tandis que la première valeur de zone d'activation est plus petite que la seconde valeur de zone d'activation.

**9.** Système selon la revendication 1, dans lequel le paramètre d'importance est déterminé en appliquant une fonction non linéaire au paramètre d'activation et au paramètre de nouveauté.

**10.** Système selon l'une quelconque des revendications 1 à 9, dans lequel le critère de sélection comprend le fait que le paramètre d'importance respectif est plus grand qu'un seuil prédéterminé.

**11.** Procédé de traitement d'information cardiaque, le procédé comprenant :

la réception d'une pluralité de signaux électriques cardiaques collectés à partir d'une pluralité d'électrodes disposées à l'intérieur d'une chambre cardiaque, dans lequel les signaux de la pluralité de signaux électriques cardiaques sont acquis sur un battement cardiaque présentant une longueur de cycle ;
la génération d'une pluralité de formes d'onde d'activation sur la base de la pluralité de signaux électriques cardiaques, dans lequel chacune de la pluralité de formes d'onde d'activation est générée sur la base de déflexions de la pluralité de signaux électriques cardiaques par rapport à une ligne de base de signal, et dans lequel la forme d'onde d'activation comprend des valeurs de forme d'onde d'activation correspondant à des probabilités que les déflexions identifiées représentent des activations de tissu cardiaque ;
le calcul d'un paramètre d'importance pour chacun de la pluralité des signaux électriques cardiaques, dans lequel le paramètre d'importance représente une contribution d'un signal électrique cardiaque respectif à un cycle opératoire global du battement cardiaque en fonction de la longueur de cycle, dans lequel le paramètre d'importance est calculé sur la base d'un paramètre d'activation et d'un paramètre de nouveauté, dans lequel le paramètre d'activation représente une première contribution du signal électrique cardiaque respectif à une zone d'activation, dans lequel le paramètre de nouveauté représente une seconde contribution du signal électrique cardiaque respectif à l'extérieur de la zone d'activation, et dans lequel le paramètre d'activation et la zone d'activation sont déterminés sur la base des valeurs de forme d'onde d'activation ; et
la facilitation de la présentation, sur un dispositif d'affichage, d'une représentation graphique de signaux électriques cardiaques sélectionnés, dans lequel chacun des signaux électriques cardiaques sélectionnés satisfait un critère de sélection sur la base d'un paramètre d'importance respectif.

FIG. 1

FIG. 2

EP 4 221 594 B1

300A

310A — receive a plurality of cardiac electrical signals collected from a plurality of electrodes

320A — calculate an importance metric for each of the plurality of cardiac electrical signals

330A — select cardiac electrical signals based on the importance metrics

340A — generate a representation of selected cardiac electrical signals

# FIG. 3A

300B

310B — receive a plurality of cardiac electrical signals collected from a plurality of electrodes

315B — generate a plurality of activation waveforms based on the plurality of cardiac electrical signals

320B — calculate an activation metric for each of the plurality of cardiac electrical signals

330B — determine an activation zone based on the activation metric

335B — calculate a novelty metric based on the activation zone for each of the plurality of cardiac electrical signals

340B — calculate an importance based on the activation metric and the novelty metric

350B — select cardiac electrical signals based on the calculated importance metrics

FIG. 3B

EP 4 221 594 B1

FIG. 4A

400

401
402
403
404
405

410

411
412
413
414
415

EP 4 221 594 B1

# FIG. 4B

410

| 411 | | 0 |
| 412 | | 0.2 |
| 413 | | 0.8 |
| 414 | | 1.0 |
| 415 | | 0.32 |

# FIG. 4C

# FIG. 4D

410

411
412
413
414
415

0
20
80
1
32

420

425

1

0

430

431
432
433
434
435

0
0
0
0
1

# FIG. 4E

FIG. 4F

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2018296108 A1
- US 2018325401 A1
- US 2012184863 A1
- US 20180296113 A
- US 8428700 B
- US 8948837 B
- US 8615287 B
- US 20150065836 A
- US 6070094 A
- US 6233491 B
- US 6735465 B